(19) Europäisches Patentamt

European Patent Office

Office européen des brevets

(11) **EP 1 522 605 A1**

(12) **EUROPEAN PATENT APPLICATION**

(43) Date of publication:
**13.04.2005 Bulletin 2005/15**

(51) Int Cl.[7]: **C23C 8/16**, C22F 1/00,
A61F 2/06, C25D 11/34

(21) Application number: **04256239.7**

(22) Date of filing: **08.10.2004**

| | |
|---|---|
| (84) Designated Contracting States:<br>**AT BE BG CH CY CZ DE DK EE ES FI FR GB GR<br>HU IE IT LI LU MC NL PL PT RO SE SI SK TR**<br>Designated Extension States:<br>**AL HR LT LV MK** | (72) Inventors:<br>• **Pelton, Alan R.**<br>  **Fremont, CA 94536 (US)**<br>• **Trepanier, Christine**<br>  **Union City, CA 94587 (US)** |
| (30) Priority: **10.10.2003 US 683377** | (74) Representative: **Fisher, Adrian John et al**<br>**CARPMAELS & RANSFORD**<br>**43-45 Bloomsbury Square**<br>**London WC1A 2RA (GB)** |
| (71) Applicant: **Nitinol Development Corporation**<br>**Fremont, California 94539 (US)** | |

(54) **Medical devices, particularly stents, and methods for their manufacture**

(57)    A medical device which includes a component formed from an alloy which contains at least about 40% Ni by weight. The alloy in a 10 nm deep surface region of the component contains not more than about 10% Ni by weight. The Ni content in that surface region can be reduced by polishing and oxidizing treatment such as (a) exposure to superheated steam, or (b) immersion in a chemical solution, or (c) an electrochemical treatment, using the device as the anode in a solution bath with a current running therethrough.

EP 1 522 605 A1

## Description

## Cross Reference to Related Appliations

This application is a continuation-in-part of Serial No. 09/760,595 filed January 16, 2001, now herewith abandoned.

## Fields of the Invention

This invention relates to medical devices and to a method of making medical devices. More particularly, this invention relates to stents and a method for making stents. Most particularly, this invention relates to self-expanding Ni-Ti stents and methods for making such stents.

## Background of the Invention

The use of nickel containing alloys in medical devices is well established. Examples of such alloys are nickel-titanium-based alloys, which are used because of their ability to exhibit shape memory properties associated with transformations between the martensitic and austenitic phases. These properties include thermally induced changes in configuration in which an article is first deformed from a heat-stable configuration to a heat-unstable configuration while the alloy is in its martensitic phase. Subsequent exposure to increased temperature results in a change in configuration from the heat-unstable configuration towards the original heat-stable configuration as the alloy reverts from its martensite phase to its austenite phase.

Nitinol is used for several medical implant devices, such as self-expanding stents. Although several studies have demonstrated good corrosion resistance and biocompatibility for Nitinol, recent studies have shown that in some cases Nitinol implants can corrode *in vivo* and release high nickel content. It has been shown that Nitinol corrosion resistance can be significantly improved by surface treatments such as electropolishing. Electropolishing of Nitinol forms a protective uniform titanium oxide passive layer that protects the base material from corrosion.

Shape memory alloys can also exhibit enhanced elastic properties compared with materials that do not exhibit martensite-austenite transformations. The nature of the superelastic transformations of shape memory alloys is discussed in "Engineering Aspects of Shape Memory Alloys", T. W. Duerig *et al*, 370, Butterworth - Heinemann (1990). Subject matter disclosed in that document is incorporated herein by reference. A principal transformation of shape memory alloys involves an initial increase in strain, approximately linearly with stress. This behavior is reversible, and corresponds to conventional elastic deformation. Subsequent increases in strain are accompanied by little or no increase in stress, over a limited range of strain to the end of the "loading plateau". The inflection point on a stress v. strain graph defines the loading plateau stress. Subsequent increases in strain are accompanied by increases in stress. Upon unloading, there is a decline in stress with reducing strain to the start of the "unloading plateau" evidenced by the existence of an inflection point along which stress changes little with reducing strain. At the end of the unloading plateau, stress reduces, with reducing strain. The inflection point on the stress v. graph also defines the unloading plateau stress. Any residual strain after unloading to zero stress is the "permanent set" of the sample. Characteristics of this deformation, the loading plateau, the unloading plateau, the elastic modulus, the plateau length and the permanent set (defined with respect to a specific total deformation) are established, and are defined in, for example, "Engineering Aspects of Shape Memory Alloys", *supra* at 376.

Many Ni-Ti alloys are considered biocompatible. However, it can be desirable for some applications to use an alloy in which the nickel content is outside the range considered acceptable for *in vivo* use, in particular to achieve a desired physical behavior in the alloy. One recently employed method to obtain a low-Ni, biocompatible surface is to use electrochemical methods. One particular concept described by this invention is to disclose alternative means to simultaneously decrease Ni content and increase biocompatibility in Ni-Ti alloys.

This invention explores the phase transformations of oxide formation in NiTi, and its effects on corrosion resistance. Electropolished Ni-50.8 at% Ti wires were heat treated between 400 and 1000°C for 3 to 300 minutes in air. Surface analytical techniques were used to characterize the thickness, composition and phase distribution of the oxide surface layers. The results of this study suggests that oxidation occurs as follows:

$$NiTi+O_2 \rightarrow Ni_3Ti+TiO_2 \rightarrow Ni_4Ti+TiO_2 \rightarrow Ni+TiO_2$$

Corrosion behavior of these oxidized wires with respect to the breakdown potential ($E_{bd}$) by potentiodynamic polarization tests was investigated. The $E_{bd}$ dramatically decreases from 1000mV to below -100mV vs SCE as the oxide thickness increases from less than 0.01μm to 10μm. Samples deformed up to 3% strain developed cracks in the titanium oxide layer and exposed the Ni-rich phases with a concomitant decrease in $E_{bd}$ below -100mV vs SCE.

At elevated temperatures in air, titanium reacts with oxygen to form a $TiO_2$ layer, and the structure of this oxide is important to understand the biocompatibility of the material. Since several Nitinol implants undergo several heat treatments to shape-set the devices or adjust its transformation temperatures as the final surface treatments, it is also important to assess the effect of oxidation of Nitinol on its corrosion resistance. Several authors [6-12] have studied the effects of surface treatments on the surface composition of NiTi, but the mechanisms of high temperature oxidation and the effects on corrosion are not entirely understood.

Therefore, the goal of this invention was to better understand the oxidation of Nitinol and determine the effect this oxidation has on corrosion resistance. Furthermore, since most implants are used under stress/strain conditions, the influence of strain on the corrosion resistance of oxidized nitinol was also assessed.

## Summary of the Invention

The present invention provides medical devices whose biocompatibility in relation to nickel content is improved.

Accordingly, in one aspect, the invention provides a medical device which includes a component formed from an alloy which contains at least about 40% Ni, the alloy in a 10 nm deep surface region of the component containing not more than about 10% Ni.

It has been found that the reduction of the nickel content of the alloy in a surface region of the Ni-alloy component can make the device of the invention more easily accepted for *in vivo* use by reducing the risk of toxic or carcinogenic side effects and by preventing its physical degradation. Preferably, the Ni content in the said surface region is reduced to not more than about 5%, more preferably not more than about 3%, especially not more than about 1.5%.

Preferably, the alloy from which the component is formed is a Ni-Ti based alloy, for example a Ni-Ti binary alloy. However, the alloy can contain more than two elements, for example a ternary alloy or a quaternary alloy. Examples of elements that can be included in a Ni-Ti based alloy include Fe, Cu, Co, Zr, Hf, B and Nb.

Preferably, the alloy contains at least about 48% Ni, more preferably at least about 50%. An example of a particularly preferred alloy is a binary alloy containing about 50.8% Ni.

The Ni content in the surface region can be determined by known spectroscopic techniques such as Auger spectroscopy, X-ray Photoelectron Spectroscopy (XPS) or Secondary Ion Mass spectroscopy (SIMS). The content is measured in a 10 nm deep layer over the area in which the component has been treated.

In another aspect, the invention provides a method of making a medical device comprising a component formed from an alloy which contains nickel, which includes the step of exposing the component in a surface region thereof to a treatment which causes the Ni content of the alloy in that region to be reduced compared with that in the remainder of the component.

## Brief Description of the Drawings

The invention will be better understood by reference to the drawings, in which:

Figure 1 is a perspective view of a stent made according to the process described herein. (A stent 10 which may be formed according to the described invention is seen in Figure 1.)

Figures 2-9 are various test results obtained as a consequence of the work preformed in this invention.

## Detailed Description of the Invention

The Ni content of the alloy in the said surface region can be reduced by specialized oxidizing treatments. Whereas investigators have tried to improve corrosion resistance by thermal oxidation heat treatments, there has been little success. Examples of treatments to which the component can be exposed include exposure to a chemical solution, exposure to superheated steam, and an electrochemical treatment. Such treatments can cause alloy elements other than nickel to be oxidized, to form an oxide layer on the component. Simultaneously, these treatments will effectively remove surface Ni atoms, thereby promoting the oxidation of Ti. For example, treatment of a Ni-Ti alloy can result in the formation of a surface layer of $TiO_2$.

A treatment which involves exposure to superheated steam will preferably involve exposure for at least about 1.5 hours, preferably at least about 3 hours, more preferably at least about 5 hours. Preferably, the steam is heated to at least about 120°C, more preferably at least about 150°C.

In electrochemical treatments of the alloy, the component is preferably included in an electrochemical system as the anode.

In chemical treatments of the alloy, the component is preferably immersed in acidic or basic solutions to modify the surface chemistry.

The Ni alloy component of the device is preferably treated so that it exhibits shape memory properties making it suitable for the particular application for the device. For example, the component can be treated so that it exhibits a thermally induced change in configuration as a result of a change in phase between austenite and martensite phases due to a change in temperature. For many applications, the component will be treated so that it exhibits enhanced elastic properties such as those referred to as "superelastic" properties.

The medical device of the invention can be designed for any of a large number of applications. A particularly preferred device is a stent formed from an alloy, which has been treated so that it exhibits enhanced elastic properties. A stent can be inserted into a lumen while constrained in a transversely compressed configuration, and then allowed to expand so that it contacts the wall of the lumen to support it, and in some applications, also radially forced outwardly. The stent is left *in situ* in the lumen to provide this support to the lumen. The reduction of the risk of complications due to adverse biocompatibility reactions that can be achieved by the present invention gives rise to particular advantages in a device that is used in this way.

Ni-Ti alloys, containing between 50% and 60% nickel by weight, are currently of great interest to the medical industry due to their superelastic and shape memory properties. These properties provide value for the design of various medical implants, such as stents, suture and bone anchors, archwire, and orthopaedic devices. As is well understood, implants made from these alloys require highly biocompatible surface finishes. Among the requirements of high biocompatibility are low uniform corrosion rates, high resistance to localized corrosion, low toxicity, and low thrombogenicity.

Unalloyed nickel is considered a toxic and carcinogenic substance. While NiTi alloys contain high nickel content, such a characteristic does not result in toxicity *per se.* In order to be toxic, nickel must be released to the environment of the device through corrosion processes. Thrombogenicity is a complicated matter, depending on the design of the device, the environment, surface chemistry and surface roughness.

The process according to the current invention describes a process to produce a very smooth and Ni-free surface on Ni-Ti. "Ni-free" in this case, is defined as less than 5% nickel in the top 10nm of the material. The process consists of two steps: polishing and oxidizing.

Ideally, the polishing process should be a polishing step that removes the existing oxide layer and leaves a surface finish of RMS 2 or better. One preferred method would be to electropolish in acidic solutions at temperatures below room temperature. These colder temperature processes result in a lower material removal rate. Lower material removal rates are more conducive to polishing complex parts with small cross sectional areas. (Larger material removal rates dictate larger currents. These cannot be uniformly carried through small cross sectional areas without excessive heating.) Polishing may be improved by preceding treatments to remove oxide layers, such as mechanical polishing and/or chemical etching.

The oxidation process can consist of a steam sterilization treatment, or better a surface treatment in a chemical solution, or even better an anodizing process. All these processes remove the Nickel from the surface and leave behind a surface film of Rutile ($TiO_2$). Immersion of the Ni-Ti component in an acidic or basic chemical solution will selectively remove the existing surface layer and promote the formation of $TiO_2$. Anodizing appears to significantly reduce the corrosion current in the passive regime and increase the corrosion potential toward more noble values.

The present invention can perhaps best be understood by reference to an example of a stent made according to the disclosed processes.

## EXAMPLE 1

The invention is described by way of a stent is made from a tube of a Ni-Ti binary alloy.

The tube from which the stent is formed is made from a Ni-Ti binary alloy which contains 50.8% Ni by weight. The tube has an external diameter of about 3 mm and a wall thickness of about 0.4 mm. Any conventional tube forming techniques, such as drawing, etching, etc, may be used to form the tube.

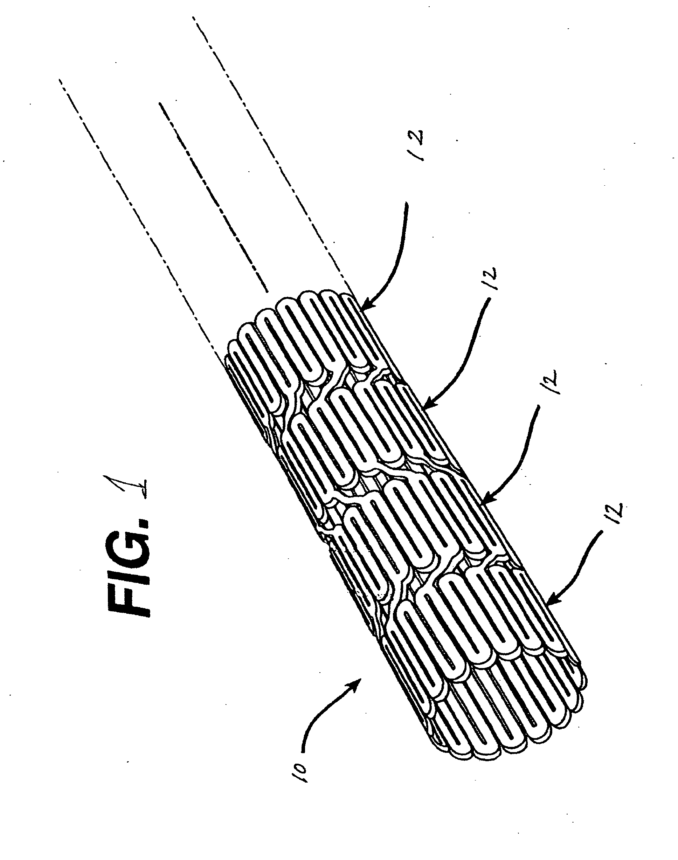

A pattern is cut into the tube so that it adopts a configuration similar to that described in Figure 1, in which there are slots 12 cut out of the tube. Conventional etching and laser cutting techniques may be used to create slots 12 into the tube, so that it forms stent 10. These slots 12 enable the radial dimension of the tube to be changed by causing the struts 14 to deform relative to one another. (It is also well understood that a segment of wire may be formed into slots 12, so that the effect of the expansion is identical to the slots formed by other processes.) Other suitable configurations of stents will be apparent; for example, stents such as the Palmaz® stent, Palmaz-Schatz® stent, Crown® stent, and Bx Velocity® stent, all made by the parent company of the present assignee, and incorporated herein by reference, are all suitable designs of stents for the herein described process. Suitable cutting techniques include laser machining (for example using a YAG laser), electric discharge machining, chemical etching and machining.

The stent is treated so that the alloy exhibits superelastic properties by a process that includes a succession of cold working and heat treatment steps (such as those found in Duerig et al., US Patent No. 5,843,244. owned by a common assignee and incorporated herein by reference.) The stent is cold worked by fitting it onto a succession of mandrels of increasing sizes. The stent is heat treated after each cold working step by exposing it to an elevated temperature (that is below the re-crystallization temperature of the alloy) while it is constrained in the configuration resulting from the cold work. A suitable heat treatment temperature is in the range 400 °C to 450 °C. A succession of cold work and heat treatment steps can be used to impart an appropriate amount of cold work to the stent, which could result in a permanent deformation if carried out in a single step.

After the cold work and heat treatment steps, the stent has superelastic properties so that it can be deformed inwardly towards the configuration as cut, and will then recover elastically towards the configuration from which it was deformed inwardly.

The stent 10 could then be electropolished at temperatures below 20°C using methanol-sulfuric acid solutions. If necessary, the stent 10 may be "primed" for polishing, by using prior treatments to remove oxide layers, such as mechanical methods (e.g., grit blasting) and/or chemical etching.

The Ni content in the alloy in a surface region of the stent is reduced by an oxidizing treatment involving exposure to superheated steam at 150 °C for 12 hours. The stent surface resulting from this treatment contains $TiO_2$. From Auger spectroscopy, the Ni content in a surface region 10 nm deep has been found to be less than 2% by weight.

Further electromechanical methods of polishing medical devices according to this invention are certainly possible and even likely. For instance, the sulfuric acid bath can be adequately substituted with other acid-based solutions, such as $HNO_3$, perchloric acid, etc. Basically, the concept applied by this step is to place the medical device into the solution bath, apply a potential, and use the device as an anode in the process. Under proper conditions, electropolishing selectively removes base material, which may contain contaminants, and allows re-growth of the passive oxide on the device surface with a low Nickel content contained thereon.

The electromechanical methods described above may further be substituted with purely chemical methods. For instance, one may use one of the following solutions:

Acidic, e.g., 10% to 50% $HNO_3$ (preferably 20% to 40% $HNO_3$; more preferably 30% $HNO_3$ - water solution);

Neutral, e.g., saline-based, such as 0.2% to 5% NaCl, preferably 0.5% to 1.5% NaCl, more preferably 0.9% NaCl - water solution;

Basic, e.g., NaOH solutions.

The purpose of these solutions is to provide a chemical environment to "leach" excess Nickel and other contaminants from the existing oxide surface and allow growth of a more passive ($TiO_2$) oxide layer. Again, these surfaces possess lower Nickel content than the levels present in typical NiTi medical devices.

In addition, the step may include anodizing. This includes exposure of the medical device to a chemical solution (acidic, neutral or basic) with an appropriately applied potential in order to grow a more stable passive oxide layer. For example, one procedure would be to immerse the NiTi medical device in a saline-based solution, and hold the potential at about 200mV to 1000mV, preferably 300mV to 700mV, more preferably 500mV for 0.1 to 10 hours, preferably 0.2 to 2 hours, more preferably 0.5 to 1 hour.

**EXAMPLE 2** - Oxidation of Nitinol and its Effect on Corrosion Resistance

Oxidation: Three mm diameter Ni-50.8 at 5 Ti wire was annealed at 1000°C for 30 minutes, centerless ground to remove the resultant oxide scale, and Electropolished. The wires were subsequently oxidized in an air furnace at 400 ti 1000°C in 100°C increments for 3, 10, 30, 100, and 300 minutes, which encompass typical Nitinol processing conditions. Auger Electron Spectroscoopy (AES), Focused Ion Beam (FIB), JEOL JSM-5600 Scanning Electron Microscope (SEM), and Oxford Instruments Model 6587 Energy Dispersive X-Ray Spectroscopy (EDXS) were used to characterize the thickness and composition of the oxide layer(s).

Corrosion Testing: In accordance with ASTM F2129, an EG&G Princeton Applied Research otentiostat model 273A was used to conduct the potentiodynamic polarization corrosion tests. The tests were conducted in Hank's simulated physiological solution at an initial pH of 7.4±0.1. The solution was maintained at 37±1°C using a water bath.

**Results and Discussion**

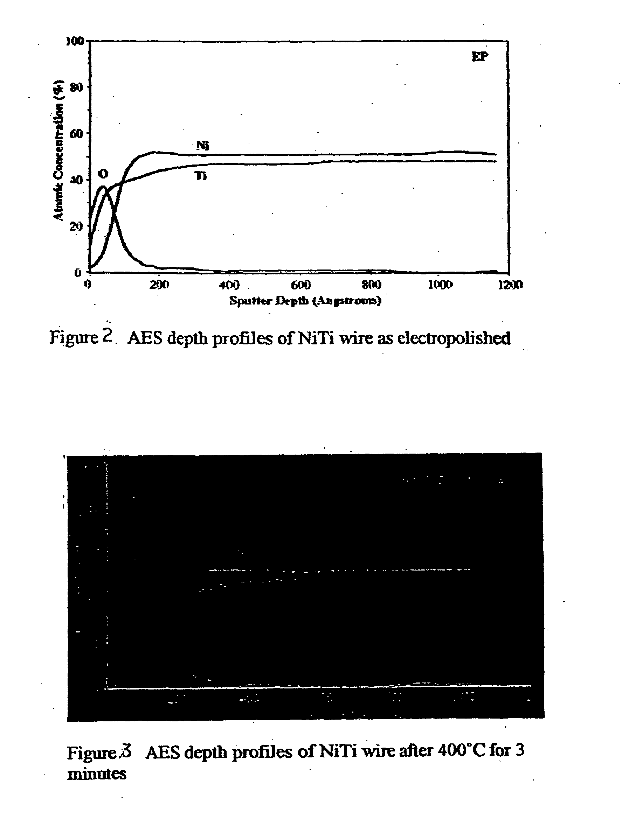

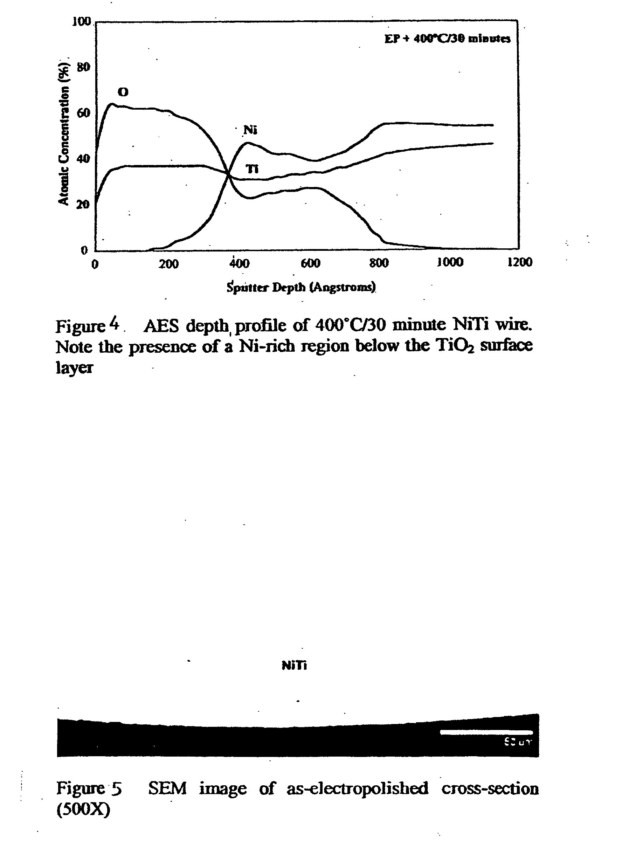

Oxidation Growth and Composition: No visible oxide was observed by SEM for samples at lower temperatures (≤600°C) and shorter times (≤30minutes) at 700°C. These samples were analyzed with AES and/or FIB to determine the oxide thickness and composition. Figures 2-3 show the AES depth profile of the Electropolished and 400°C/3 min samples, with a titanium oxide thickness of 110 Å and 200 Å, respectively. After 30 minutes at 400°C, AES revealed a nickel-rich region beneath the outer $TiO_2$ layer (Figure 4). The remaining samples analyzed with AES showed a more pronounced nickel-rich region below the surface $TiO_2$ layer.

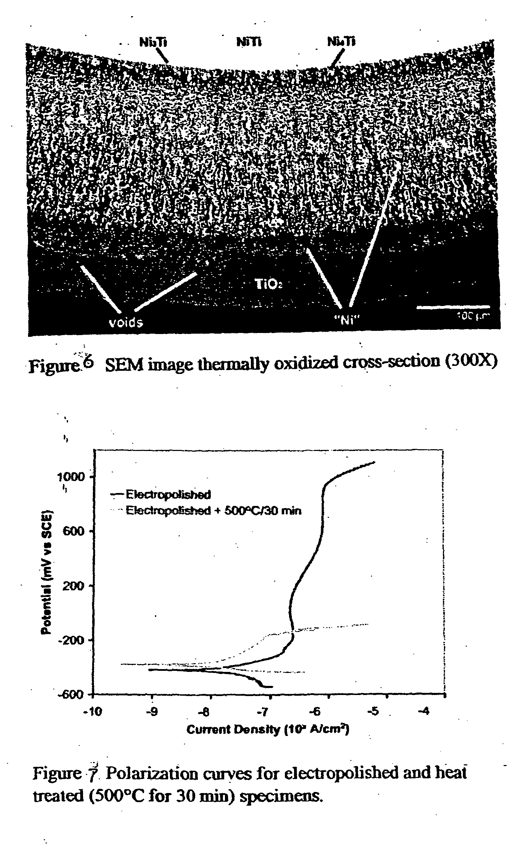

Figures 5 and 6 show metallographic cross-sections of the as-electropolished wire and thermally oxidized wire, respectively. The bright interfacial region between the base NiTi and the surface titanium oxide observed for the oxidized samples was analyzed by EDXS to be 75at% Ni and 25at% Ti, consistent with $Ni_3Ti$ (while sublayer); EDXS also confirms the presence of $TiO_2$ (dark gray). Small $Ni_3Ti$ finger-like projections emerge from the nickel-rich layer and appear to form islands in the oxide. A comprehensive EDXS analysis indicates that the Ni content of the phases increases with increasing distance from the NiTi interface. The $Ni_3Ti$ interfacial layer transforms to $Ni_4Ti$ (80at% Ni), whereas the islands become nearly pure Ni (approximately 92 at% Ni). This composition transition indicates that with increasing time at temperature, the Ni3Ti sublayer becomes Ti-depleted as the Ti reacts with oxygen, leaving behind nearly pure Ni.

Corrosion: The corrosion resistance of the oxidized Nitinol specimens depends on the time and temperature of the heat treatment. As shown in Figure 7, the Electropolished sample has a very high breakdown potential (approximately 1000 mV), which indicates extremely good corrosion resistance. Other specimens, however, showed breakdown potentials as low as -140 mV vs SCE, such as specimens heat treated at 500°C for 30 minutes.

Deformation of the Electropolished and thermally oxidized wires (3% strain) resulted in severe cracking of the oxide layer, which also greatly influenced the corrosion test results. For example, the breakdown potential of the Nitinol specimens heat treated at 400°C for 10 min. decreased from 1030 mV vs SCE to 417 mV vs SCE (Figure 7).

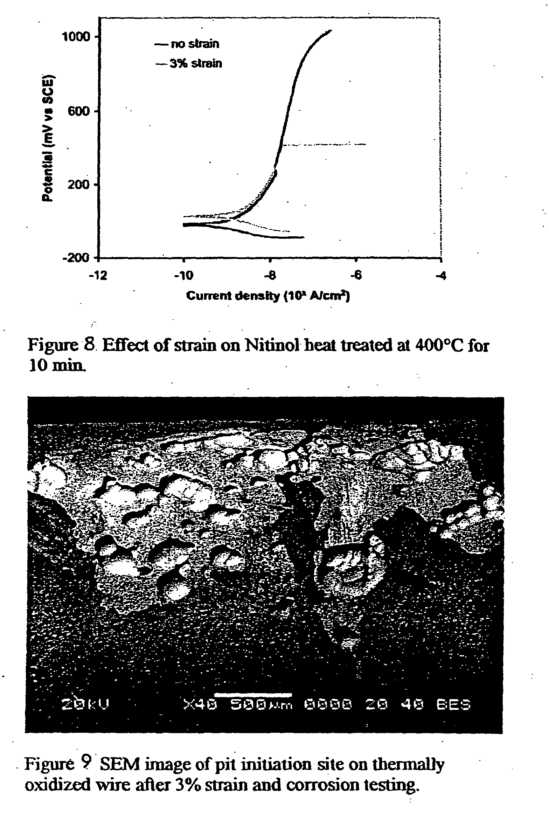

The presence of nickel-rich phases in the thermally grown oxide layer of Nitinol can be detrimental to the corrosion resistance of the material. The significant decrease in the corrosion resistance of the specimens with thermally grown oxide layers is due to defects or breaks in the protective titanium oxide layer. Deformation of the specimens up to 3% strain had a similar effect. SEM and EDXS analyses clearly showed that localized corrosion (pitting) initiated in the Ni-rich phase as seen in Figure 9, where the nickel-rich phase appears light gray while the titanium oxide layer appears darker gray.

Furthermore, it is inevitable that Nitinol implants will undergo significant deformation during their use from either shape memory or from superelasticity. For example, it is also not unusual for self-expandable Nitinol stents to be deformed up to 8% strain when constrained in the delivery system before their deployment. As was shown in the present study, when oxidized Nitinol is deformed, the oxide layer may crack and expose the nickel-rich phases, thereby lowering the corrosion resistance of the material. Since the oxide layer on Nitinol is not superelastic, a thin oxide layer is preferable since it can flex and sustain the large deformations of the underlying Nitinol material without cracking.

Nitinol products (wire, tubes, sheet, strip, and components made from these raw materials) go through several heat treatments in the temperature range of approximately 400°C to 1000°C during processing. Under these conditions, the corrosion resistance is shown to be poor due to the thermal growth of Nickel-rich phases and porous Titanium oxide. Therefore, it is essential that the implants undergo a surface treatment to remove the thick oxide layer and passivate the surface. During chemical polishing and electropolishing, process nickel is preferentially removed. These effects were dramatically demonstrated in a recent publication that showed that explanted Nitinol stents had failed *in vivo* due to severe corrosion. These stents had a thermally grown oxide layer which did not provide adequate corrosion resistance in the body. Properly passivated stents, however, were explanted with no signs of corrosion.

Of course, it is to be understood that a broad range of equivalent steps are quite possible by which to make stents according to the present invention. For instance, the rates of polishing may be adjusted depending on the desired surface finish. Also, the length of time to heat the stent with superheated steam may be adjusted depending on the size of the stent, or the number of stents heated in a batch. Furthermore, for chemical treatments, the volume of solution and duration of exposure will be adjusted according to the number of stents and the prior processing steps.

This invention demonstrates that the phase transformation for oxidized NiTi. Electropolished wires are characterized by a thin ($\sim 0.01 \mu m$) titanium oxide layer. The thickness of the oxide layer increases with increasing oxidation time at temperature between 400°C and 1000°C. A Ni-rich layer is observed at the interface between NiTi and the thermal $TiO_2$. The oxidation reactions for these samples appear to proceed as follows:

$$NiTi + O_2 \rightarrow Ni_3Ti + TiO_2 \rightarrow Ni_4Ti + TiO_2 \rightarrow Ni + TiO_2$$

The breakdown potential dramatically decreases from 1000mV to below -100mV vs SCE as the oxide thickness increases (due to higher temperatures and longer times). The significant decrease in the corrosion resistance of the specimens with thermal oxide layers is due to defects or breaks in the oxide layer. These superficial cracks expose Ni-rich phases that grow during the thermal oxidation of Nitinol. Samples deformed up to 3% strain in bending developed cracks in the oxide layer and exposed the Ni-rich phases with a concomitant decrease in breakdown potential to below - 100mV vs SCE. Properly passivated samples, however, demonstrated excellent corrosion resistance even after 3% strain.

## Claims

1. A method for forming a medical device providing a medical device which includes a component formed from an alloy which contains at least about 40% at Ni;

   oxidating the surface of the device wherein the oxidating step produces an oxide layer on the surface of the stent of up to 10nm depth wherein said surface region contains not more than about 5% Ni, and a Ni rich layer below the layer.

2. A device as claimed in claim 1, in which the alloy in the said surface region contains not more than about 3% Ni.

3. A device as claimed in claim 1, in which the alloy has been subjected to polishing and oxidizing treatment on said surface region.

4. A device as claimed in claim 3, in which the said polishing treatment comprises an electrochemical or mechanical treatment.

5. A device as claimed in claim 3, in which the said oxidizing treatment comprises at the steps of exposure to superheated steam, a chemical treatment and an electrochemical treatment.

6. A device as claimed in claim 1, in which the alloy is a Ni-Ti based alloy.

7. The device of claim 5, in which the said electrochemical oxidizing treatment comprises anodizing in a acidic, neutral or basic solution.

8. A device as claimed in claim 1, in which the device has been treated so that it exhibits superelastic properties.

9. A device as claimed in claim 1, in which the alloy contains at least about 48% at Ni.

10. A device as claimed in claim 9, in which the alloy contains at least about 50% at Ni.

11. A. device as claimed in claim 1, in the form of a stent.

FIG. 1

Figure 2. AES depth profiles of NiTi wire as electropolished

Figure 3    AES depth profiles of NiTi wire after 400°C for 3 minutes

Figure 4. AES depth profile of 400°C/30 minute NiTi wire. Note the presence of a Ni-rich region below the TiO₂ surface layer

Figure 5  SEM image of as-electropolished cross-section (500X)

Figure 6  SEM image thermally oxidized cross-section (300X)

Figure 7. Polarization curves for electropolished and heat treated (500°C for 30 min) specimens.

Figure 8. Effect of strain on Nitinol heat treated at 400°C for 10 min.

Figure 9 SEM image of pit initiation site on thermally oxidized wire after 3% strain and corrosion testing.

**European Patent Office**

## EUROPEAN SEARCH REPORT

Application Number

EP 04 25 6239

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 222 901 A (NITINOL DEV CORP) 17 July 2002 (2002-07-17) * claims 1-14 * | 1-11 | C23C8/16 C22F1/00 A61F2/06 C25D11/34 |
| A | US 5 413 642 A (ALGER DONALD L) 9 May 1995 (1995-05-09) * abstract * | 1 | |
| A | TREPANIER ET AL: "Improvement of the corrosion resistance of NiTi stents by surface treatments" CA, vol. 126, no. 319897, 1997, XP002200502 * abstract * | 1,3-6,8, 11 | |
| A | TREPANIER ET AL: "Effect of modification of oxide layer on NiTi stent corrosion resistance" CA, vol. 130, no. 129906, 1997, XP002200503 * abstract * | 1,3-6,8, 11 | TECHNICAL FIELDS SEARCHED (Int.Cl.7) C23C C25D C22F |

The present search report has been drawn up for all claims

| Place of search | Date of completion of the search | Examiner |
|---|---|---|
| The Hague | 30 November 2004 | Elsen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 04 25 6239

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2004

| Patent document cited in search report | | Publication date | Patent family member(s) | | Publication date |
|---|---|---|---|---|---|
| EP 1222901 | A | 17-07-2002 | US<br>AU<br>CA<br>EP<br>US | 2002092583 A1<br>1020002 A<br>2367790 A1<br>1222901 A2<br>2004117001 A1 | 18-07-2002<br>18-07-2002<br>16-07-2002<br>17-07-2002<br>17-06-2004 |
| US 5413642 | A | 09-05-1995 | US | 5599404 A | 04-02-1997 |

EPO FORM P0459

For more details about this annex : see Official Journal of the European Patent Office, No. 12/82